# EUROPEAN PATENT APPLICATION

(11) **EP 4 465 027 A1**
(43) Date of publication of application: **20.11.2024**
(21) Application number: 22920549.7
(22) Date of filing: 14.12.2022
(51) Int. Cl.: G01N 23/04, G01N 23/18

(54) **INFORMATION PROCESSING DEVICE, INFORMATION PROCESSING METHOD, AND PROGRAM**

(30) Priority: 14.01.2022 JP 2022004405
(71) Applicant: FUJIFILM Corporation, Tokyo 106-8620 (JP)
(72) Inventor: TAIRA, Mitsutoshi, Tokyo 106-8620 (JP); YONAHA, Makoto, Tokyo 106-8620 (JP); TANAKA, Eiichi, Tokyo 106-8620 (JP); INAGI, Seiya, Tokyo 106-8620 (JP); IKEDA, Haruka, Tokyo 106-8620 (JP)
(74) Representative: Meissner Bolte Partnerschaft mbB
(86) International application number: PCT/JP2022/045979
(87) International publication number: WO 2023/136031

(57) **Abstract**

Provided are an information processing apparatus, an information processing method, and a program capable of efficiently and accurately checking an occurrence distribution of discontinuities. An information processing apparatus (10) is an information processing apparatus (10) including a processor (14), in which the processor (14) is configured to: receive a display selection instruction from an operation unit (24) operated by a user; acquire discontinuity information that is information related to a discontinuity of a component and includes positional information of the discontinuity on the component; acquire an occurrence position distribution of the discontinuity based on the discontinuity information; and display the occurrence position distribution on an image of the component in response to the display selection instruction.

## Description

### BACKGROUND OF THE INVENTION

### 1. Field of the Invention

The present invention relates to an information processing apparatus, an information processing method, and a program.

### 2. Description of the Related Art

An aeronautical component such as a turbine blade requires a high quality in manufacturing thereof. In addition, manufacturing costs of an aeronautical component are often high due to use of expensive materials or many complicated processing steps involved. In particular, in a case where a defect is found inside a cast component, it is often impossible to repair the defect. Such a component that cannot be repaired is discarded, which causes a large loss. Therefore, in a case of manufacturing a component having a casting process, which requires high-accuracy manufacturing, such as an aeronautical component, it is required to improve the yield.

In addition, in the related art, a defect inspection of a completed component or the like has been performed using a radiation image. In the defect inspection, a discontinuity that is a defect candidate is detected from the radiation image, and a detection result is displayed on a display unit such as a monitor.

WO2017/130550A discloses a technique for selecting a thickness and a size and displaying a discontinuity (defect candidate) according to the selected thickness and size.

### SUMMARY OF THE INVENTION

Here, it is difficult to predict an occurrence of a defect in a component, and a cause analysis or consideration of countermeasures is often performed after the defect has occurred. There is also an attempt to acquire manufacturing environment data such as a temperature and vibration in a manufacturing process by utilizing the Internet of Things (IoT) and predict the occurrence of the defect. However, since a change inside a component cannot be captured only with such indirect data, an accuracy of defect prediction is low in a current situation.

Therefore, it is necessary to efficiently and accurately ascertain at which location and how many defects or discontinuities (defect candidates) occur in the completed component. As described above, an ascertained occurrence distribution of the discontinuities can be used to accurately ascertain a state of a manufacturing component and to improve a manufacturing process or a design process.

The present invention has been made in view of such circumstances, and an object thereof is to provide an information processing apparatus, an information processing method, and a program capable of efficiently and accurately checking an occurrence distribution of discontinuities.

According to one aspect of the present invention for achieving the above object, there is provided an information processing apparatus comprising a processor, in which the processor is configured to: receive a display selection instruction from an operation unit operated by a user; acquire discontinuity information that is information related to a discontinuity of a component and includes positional information of the discontinuity on the component; acquire an occurrence position distribution of the discontinuity based on the discontinuity information; and display the occurrence position distribution on an image of the component in response to the display selection instruction.

According to the present aspect, the display selection instruction that is a selection of the component to be displayed is received, and the occurrence position distribution corresponding to the display selection instruction is displayed on the image of the component. As a result, it is possible to efficiently and accurately ascertain the occurrence distribution of discontinuities.

Preferably, the display selection instruction gives an instruction for positions of arrangement of a plurality of the components, and the occurrence position distribution corresponding to the positions of the arrangement is displayed.

Preferably, the arrangement has a component in time.

Preferably, the time is related to manufacturing or inspection of the component.

Preferably, the discontinuity is detected by transmission of radiation.

Preferably, the processor is configured to display the discontinuity information in relation to the occurrence position distribution.

Preferably, the processor is configured to perform a user interface display indicating a point in time of manufacturing of the component or inspection of the component on a display unit, and the display selection instruction is received by the user interface display and is composed of the point in time of manufacturing of the component or inspection of the component.

Preferably, the processor is configured to perform a user interface display indicating a manufacturing number of the component on a display unit, and the display selection instruction is received by the user interface display and is composed of the manufacturing number.

Preferably, the processor is configured to also display the discontinuity information on the user interface display.

Preferably, the discontinuity information displayed on the user interface display is the number of discontinuities or a total area of the discontinuities.

Preferably, the discontinuity information is displayed on the user interface display according to a set reference value.

Preferably, the discontinuity information includes information on a type of the discontinuity, and the user interface display displays the discontinuity information for each type of the discontinuity.

Preferably, the display selection instruction selects a period on the user interface display, and the occurrence position distribution corresponding to the period is displayed on the image.

Preferably, the display of the occurrence position distribution is movable.

Preferably, the display of the occurrence position distribution is enlargeable or reducible.

Preferably, the image is a radiation image of the component, a plurality of the radiation images subjected to registration, or a design drawing of the component.

Preferably, the display selection instruction is a selection of the component to be displayed.

According to another aspect of the present invention, there is provided an information processing method for an information processing apparatus including a processor, the method causing the processor to execute: a step of receiving a display selection instruction from an operation unit operated by a user; a step of acquiring discontinuity information that is information related to a discontinuity of a component and includes positional information of the discontinuity on the component; a step of acquiring an occurrence position distribution of the discontinuity based on the discontinuity information; and a step of displaying the occurrence position distribution on an image of the component in response to the display selection instruction.

According to still another aspect of the present invention, there is provided a program for causing an information processing apparatus including a processor to execute an information processing method, the program causing the processor to execute: a step of receiving a display selection instruction from an operation unit operated by a user; a step of acquiring discontinuity information that is information related to a discontinuity of a component and includes positional information of the discontinuity on the component; a step of acquiring an occurrence position distribution of the discontinuity based on the discontinuity information; and a step of displaying the occurrence position distribution on an image of the component in response to the display selection instruction.

According to the present invention, since the display selection instruction that is a selection of the component to be displayed is received and the occurrence position distribution according to the display selection instruction is displayed on the image of the component, the occurrence distribution of discontinuities can be efficiently and accurately ascertained.

### BRIEF DESCRIPTION OF THE DRAWINGS

Fig. 1 is a block diagram showing a configuration example of hardware of an information processing apparatus.
Fig. 2 is a diagram showing an example of discontinuity information stored in a discontinuity information DB.
Fig. 3 is a diagram illustrating a radiation image obtained by imaging a component from which the discontinuity information shown in Fig. 2 is acquired.
Fig. 4 is a diagram showing a functional block.
Fig. 5 is a flowchart for describing an information processing method.
Fig. 6 is a diagram showing a first display mode.
Fig. 7 is a diagram showing the first display mode.
Fig. 8 is a diagram showing a second display mode.
Fig. 9 is a diagram showing the second display mode.
Fig. 10 is a diagram showing a third display mode.
Fig. 11 is a diagram showing a fourth display mode.
Fig. 12 is a diagram showing a fifth display mode.
Fig. 13 is a diagram illustrating a display mode of an occurrence position distribution.
Fig. 14 is a block diagram showing a defect inspection device that acquires the discontinuity information.
Fig. 15 is a block diagram showing an example of an image processing unit.
Fig. 16 is a block diagram showing an example of inspection object imaging data.
Fig. 17 is a block diagram showing an example of product data.
Fig. 18 is a block diagram showing an example of inspection object inspection result data.
Fig. 19 is a block diagram showing an example of an imaging system.

### DESCRIPTION OF THE PREFERRED EMBODIMENTS

Hereinafter, preferred embodiments of an information processing apparatus, an information processing method, and a program according to the present invention will be described with reference to the accompanying drawings.

### <Information Processing Apparatus>

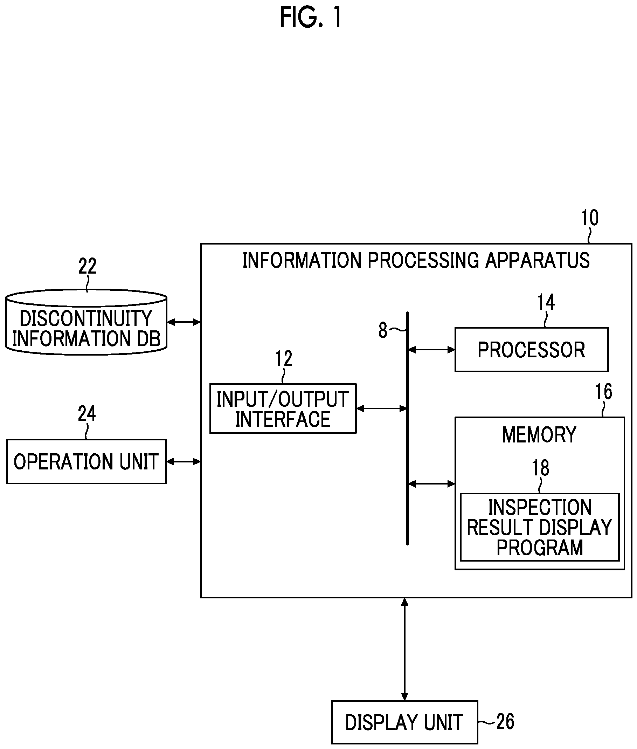

Fig. 1 is a block diagram showing a configuration example of hardware of the information processing apparatus according to the embodiment of the present invention.

An information processing apparatus 10 includes a processor 14, a memory 16 configured of a non-transitory tangible object, and an input/output interface 12.

The processor 14 is configured of a central processing unit (CPU). In addition, the processor 14 may be configured of a graphics processing unit (GPU). The processor 14 is connected to the memory 16 and the input/output interface 12 via a bus 8.

Information is input to the information processing apparatus 10 via the input/output interface 12. In addition, information is output via the input/output interface 12. For example, a discontinuity information database (DB) 22, an operation unit 24, and a display unit 26 are connected to the information processing apparatus 10 via the input/output interface 12.

The memory 16 includes a memory that is a main storage device and a storage that is an auxiliary storage device. The memory 16 may be, for example, a semiconductor memory, a hard disk drive (HDD) device, a solid state drive (SSD) device, or a combination thereof.

The memory 16 stores a program that controls the information processing apparatus 10. For example, an inspection result display program 18 for executing an inspection result display method described later is stored. The memory 16 stores a general program for operating the information processing apparatus 10.

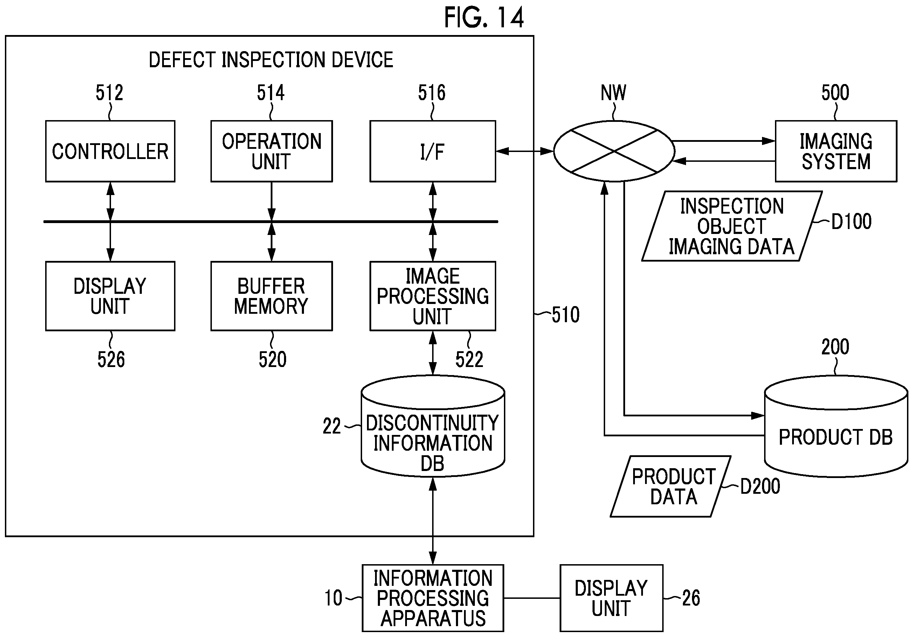

The discontinuity information DB 22 stores discontinuity information acquired (or generated) by a defect inspection device 510 (see Fig. 14). Acquisition of the discontinuity information in the defect inspection device 510 will be described later.

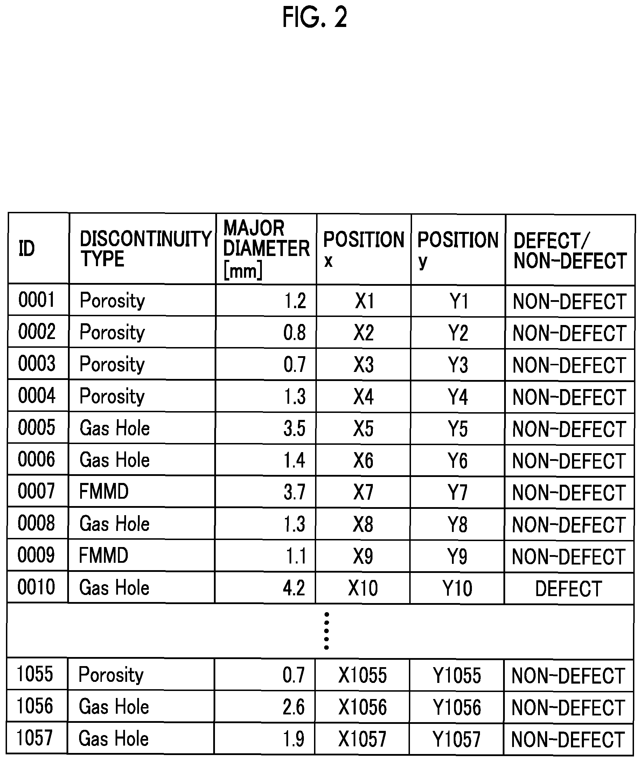

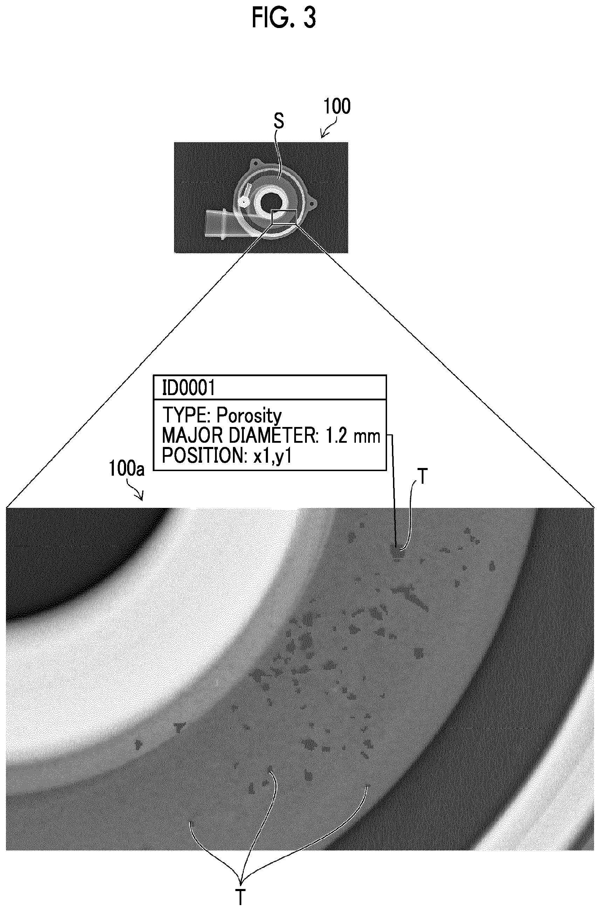

Fig. 2 is a diagram showing an example of the discontinuity information stored in the discontinuity information DB 22. In addition, Fig. 3 is a diagram illustrating a radiation image (including a transmission image and an X-ray image) 100 obtained by imaging a component S from which the discontinuity information shown in Fig. 2 is acquired. In the following description, the terms "discontinuity" and "defect" are used to be distinguished from each other. The discontinuity is detected by transmission of radiation and is detected by observing a displayed radiation image by an image processing unit 522 (see Fig. 14) or an interpreter (inspector) described later. The defect is a defect determined by the interpreter or automatically among the detected discontinuities. For example, the defect is a discontinuity having a predetermined major diameter determined as a defect.

The discontinuity information shown in Fig. 2 shows discontinuity information for one component S. The discontinuity information is composed of a discontinuity ID, a discontinuity type, a major diameter (mm), a position x, a position y, and a determination result of a defect or a non-defect. The discontinuity ID is a number assigned to each discontinuity. The discontinuity type indicates a type of the detected discontinuity. For example, Porosity, Gas Hole, Foreign Material More Density (FMMD), and the like are assigned as the type of discontinuity. Here, the Porosity means a general term for blowholes, caterpillar-like pits perforated to a surface, and the like, which has occurred in a welded metal portion after solidification due to a gas generated in a molten metal. In addition, the Gas Hole is a general term for casting defects caused by a gas included in a casting. The FMMD means incorporation of high-density foreign substances. The major diameter (mm) indicates the longest diameter of the discontinuity. The position x and the position y indicate the position of the discontinuity. The determination of the defect/non-defect indicates, for example, a determination result by a user. It should be noted that the user here refers to, for example, an interpreter or an inspector.

The discontinuity information DB 22 stores the above-described discontinuity information in association with each component.

Fig. 3 shows an example of a radiation image from which the discontinuity information shown in Fig. 2 is acquired.

Fig. 3 shows a radiation image 100 from which the discontinuity information shown in Fig. 2 is acquired and an enlarged view 100a of the radiation image 100.

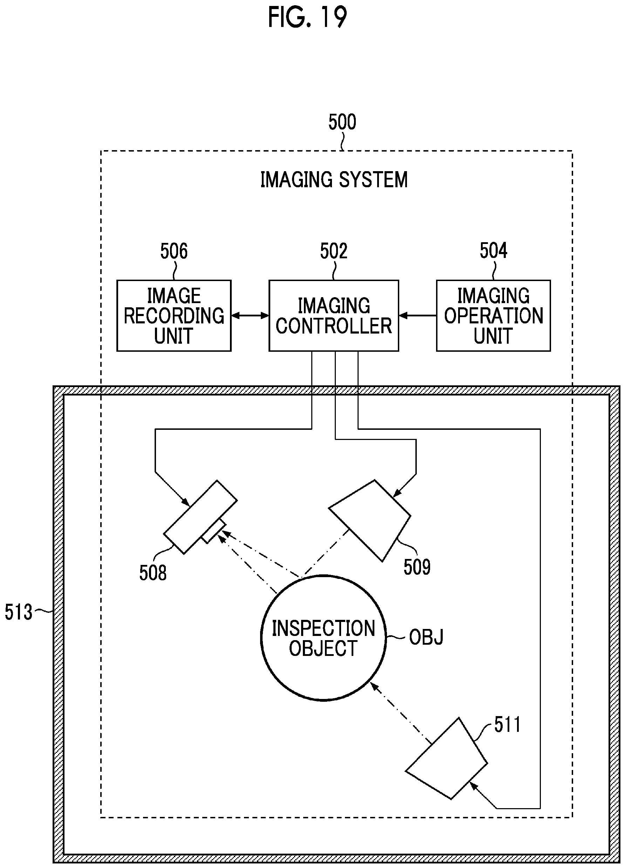

The radiation image 100 is captured by an imaging system 500 (Fig. 19) to be described later. Thereafter, the defect inspection device 510 detects a discontinuity T. As described above, each of the detected discontinuities T is given a discontinuity ID, and the type, the major diameter, the position, and the determination result of the defect or the non-defect are acquired. Fig. 3 shows a specific example of a discontinuity ID 0001.

Returning to Fig. 1, the operation unit 24 is an input device that receives an operation input from the user, and includes a keyboard for inputting characters, and a pointing device (a mouse, a trackball, or the like) for operating a pointer, an icon, or the like displayed on the display unit 26. In addition, a touch panel may be provided on a surface of the display unit 26 as the operation unit 24, instead of the means listed above or in addition to the means listed above.

The display unit 26 is a device for displaying an image. As the display unit 26, for example, a liquid crystal monitor can be used.

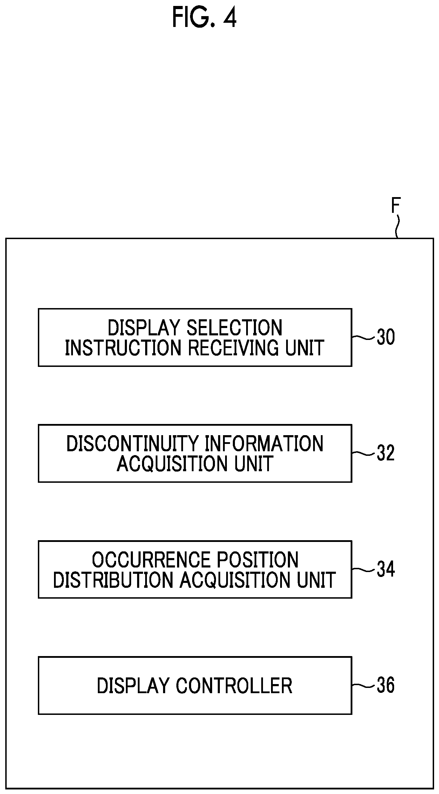

Fig. 4 is a diagram showing a functional block of a function F that is realized by the processor 14 executing the inspection result display program 18 stored in the memory 16.

The function F realized by the processor 14 executing the inspection result display program 18 comprises a display selection instruction receiving unit 30, a discontinuity information acquisition unit 32, an occurrence position distribution acquisition unit 34, and a display controller 36.

The display selection instruction receiving unit 30 receives a display selection instruction that is a selection of a component to be displayed from the operation unit 24 that is operated by the user. For example, the user selects a component to be displayed on a user interface display displayed on the display unit 26, and the display selection instruction receiving unit 30 receives the display selection instruction.

The discontinuity information acquisition unit 32 acquires discontinuity information that is information related to a discontinuity on a component and includes positional information of the discontinuity on the component. For example, the discontinuity information acquisition unit 32 acquires the discontinuity information of the component selected by the display selection instruction from the discontinuity information DB 22.

The occurrence position distribution acquisition unit 34 acquires an occurrence position distribution of the discontinuities based on the discontinuity information. For example, the occurrence position distribution acquisition unit 34 acquires the occurrence position distribution on the component for each discontinuity based on the position x and the position y of the discontinuity information. Here, by displaying the occurrence position distribution on the image of the component, the user can efficiently and accurately ascertain at which portion of the component and to what extent the discontinuities have occurred (occurrence distribution). In a case where the occurrence position distribution is displayed on the display unit 26, the discontinuity information related to the displayed occurrence position distribution may be displayed.

The display controller 36 displays the occurrence position distribution on the image of the component displayed on the display unit 26 in response to the display selection instruction. In addition, the display controller 36 displays, on the display unit 26, not only the display of the occurrence position distribution but also a user interface (a timeline bar 104 and a pointer 106) described later, a detailed information display 102, the radiation image 100, and the like.

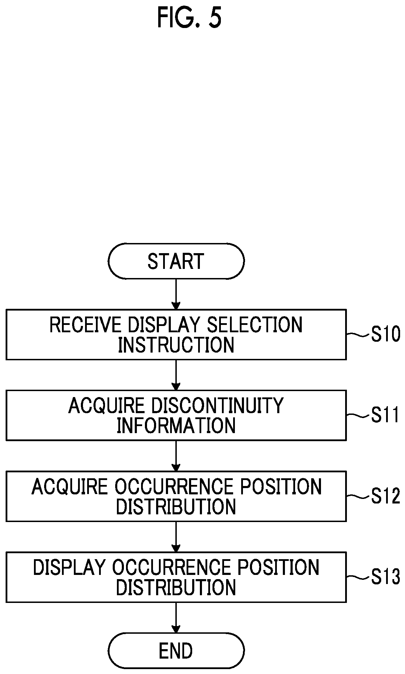

Fig. 5 is a flowchart illustrating an information processing method performed by using the information processing apparatus 10. The information processing method is performed by the processor 14 executing the inspection result display program 18 stored in the memory 16.

First, the display selection instruction receiving unit 30 receives the display selection instruction that is a selection of a component to be displayed (step S10). Next, the discontinuity information acquisition unit 32 acquires the discontinuity information of the component corresponding to the display selection instruction (step S11). Next, the occurrence position distribution acquisition unit 34 acquires the occurrence position distribution of the discontinuities based on the discontinuity information (step S12). Then, the display controller 36 displays the occurrence position distribution on the image of the component displayed on the display unit 26 (step S13).

Next, a display mode of the display unit 26, which is performed mainly by the display controller 36 of the information processing apparatus 10, will be described.

### <First Display Mode>

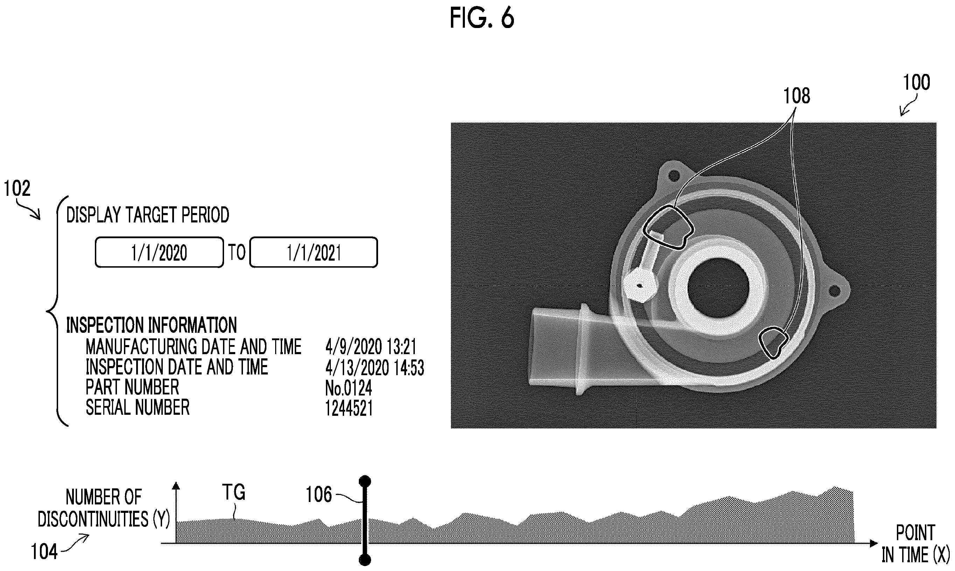

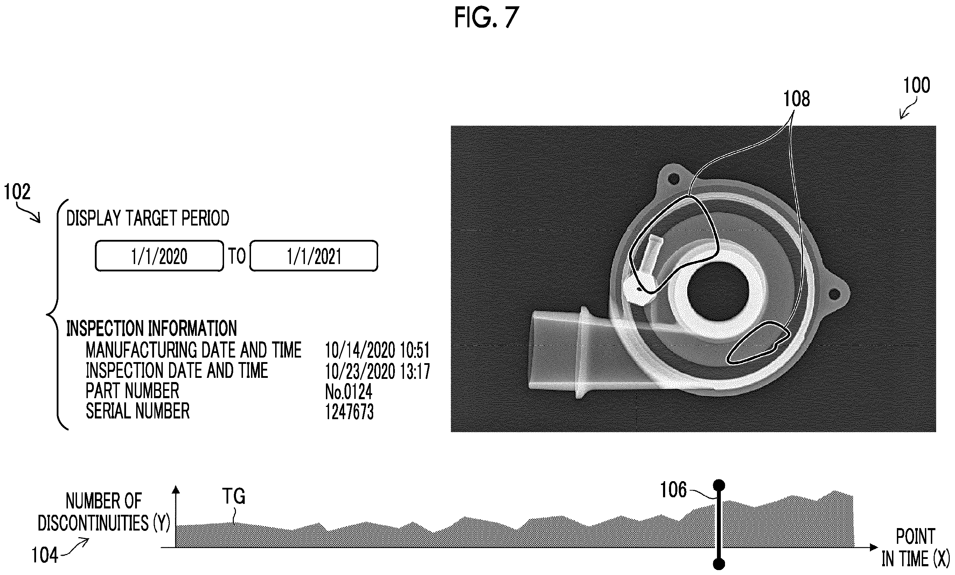

Figs. 6 and 7 are diagrams showing a first display mode.

In the first display mode, the radiation image 100, an occurrence position distribution 108 of discontinuities displayed to be superimposed on the radiation image 100, the detailed information display 102, and the timeline bar 104 having the pointer 106 are displayed on the display unit 26.

The timeline bar 104 has an X-axis and a Y-axis, and points in time are shown in time series on the X-axis, and the number of discontinuities TG is shown on the Y-axis. The number of discontinuities TG indicates the number of discontinuities on the component corresponding to a manufacturing date and time indicated on the X-axis. The displayed number of discontinuities TG is an example of the discontinuity information displayed in relation to the timeline bar 104. The discontinuity information displayed in relation to the timeline bar 104 may be a total area of the discontinuities.

The timeline bar 104 has a component in time in which manufacturing dates and times of components are arranged in time series on the display unit 26, and is an example of the user interface display. The user can select the components having different manufacturing dates and times by operating the pointer 106. In the above-described example, the timeline bar 104 in which the manufacturing dates and times of the components are arranged in time series is described, but the present invention is not limited thereto. For example, inspection points in time of the components may be arranged in time series on the X-axis of the timeline bar 104. In addition, as another example of the user interface display, a serial number bar in which serial numbers (manufacturing numbers) of components are arranged in time series may be displayed.

The user can superimpose and display the occurrence position distribution 108 of discontinuities of the component having a manufacturing date and time at a point in time at which the pointer 106 is positioned on the radiation image 100 by moving the pointer 106 along the X-axis. That is, the display selection instruction, which is the selection of the component to be displayed by the timeline bar 104 that is an example of the user interface display, is input. As described above, the display selection instruction gives an instruction for positions of the arrangement of a plurality of components. Then, the occurrence position distribution corresponding to the positions of the arrangement for which an instruction is given by the display selection instruction is displayed.

In the detailed information display 102, a display target period of the occurrence position distribution of discontinuities is shown. For example, the timeline bar 104 displays information related to the component manufactured in the display target period (from January 1, 2020 to January 1, 2021). In addition, a manufacturing date and time, an inspection date and time, a part number, and a serial number are shown as inspection information. As the inspection information, inspection information of a component corresponding to a point in time indicated by the pointer 106 is displayed on the timeline bar 104. Specifically, Fig. 6 shows an occurrence position distribution of a component having a serial number 1244521, and as shown in Fig. 7, in a case where the pointer 106 is moved, an occurrence position distribution of a component having a serial number 1247673 is shown. In the above-described example, a case where one component is selected by the pointer 106 has been described, but the present invention is not limited thereto. For example, a predetermined period may be selected on the timeline bar 104, and the occurrence position distribution 108 of a plurality of components corresponding to the period may be displayed on the radiation image 100.

As described above, according to the present display mode, the user can select the component to be displayed by the timeline bar 104 displayed on the display unit 26, and the occurrence position distribution of the discontinuities of the selected component can be displayed to be superimposed on the radiation image 100. As a result, the user can efficiently and accurately check the occurrence distribution of the discontinuities.

### <Second Display Mode>

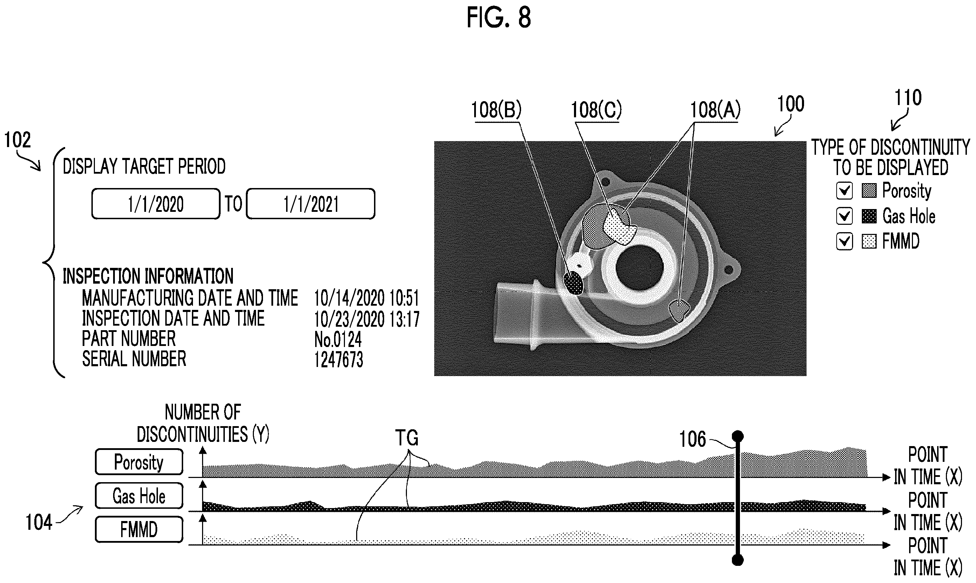

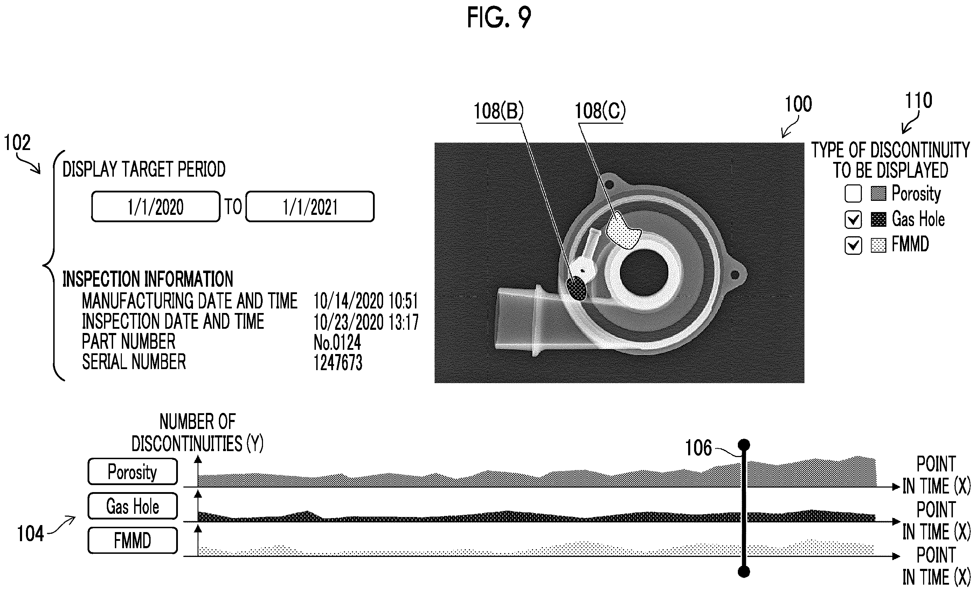

Figs. 8 and 9 are diagrams showing a second display mode. In addition, the portions already described in Figs. 6 and 7 are denoted by the same references, and the description thereof will not be repeated.

In the second display mode, the radiation image 100, occurrence position distributions 108(A), 108(B), and 108(C) of the discontinuities displayed to be superimposed on the radiation image 100, the detailed information display 102, the timeline bar 104 having the pointer 106, and a type display 110 of discontinuities to be displayed are displayed.

In the present display mode, the timeline bar 104 is displayed for each type of the discontinuity. Specifically, a timeline bar 104 related to Porosity, a timeline bar 104 related to Gas Hole, and a timeline bar 104 related to FMMD are displayed. In addition, the number of each type of the discontinuity is also displayed on the timeline bar 104 related to Porosity, the timeline bar 104 related to Gas Hole, and the timeline bar 104 related to FMMD, as described in the first display mode.

In the radiation image 100, the discontinuity occurrence position distributions 108(A), 108(B), and 108(C) related to the component having the serial number 1247673 are shown. The occurrence position distribution 108(A) shows the occurrence position distribution of Porosity, the occurrence position distribution 108(B) shows the occurrence position distribution of Gas Hole, and the occurrence position distribution 108(C) shows the occurrence position distribution of FMMD.

The type display 110 of the discontinuity to be displayed is composed of checkboxes of Porosity, Gas Hole, and FMMD. By using the checkboxes, the user can select the display of the occurrence position distributions 108(A), 108(B), and 108(C) related to Porosity, Gas Hole, and FMMD.

In Fig. 8, in the type display 110 of discontinuities to be displayed, the checkboxes of Porosity, Gas Hole, and FMMD are checked. Therefore, the occurrence position distribution 108(A), the occurrence position distribution 108(B), and the occurrence position distribution 108(C) are displayed on the radiation image 100.

Meanwhile, in the example shown in Fig. 9, the check of the checkbox of Porosity is released in the type display 110 of discontinuities to be displayed. Accordingly, the occurrence position distribution 108(A) corresponding to the Porosity is not displayed, and the occurrence position distribution 108(B) and the occurrence position distribution 108(C) corresponding to Gas Hole and FMMD are displayed.

As described above, according to the present embodiment, the occurrence position distributions 108(A), 108(B), and 108(C) can be selectively displayed for each type of the discontinuity by the type display 110 of discontinuities to be displayed. In addition, the timeline bar 104 is displayed for each type of the discontinuity. As a result, the user can efficiently and accurately check the occurrence distribution for each type of the discontinuity.

### <Third Display Mode>

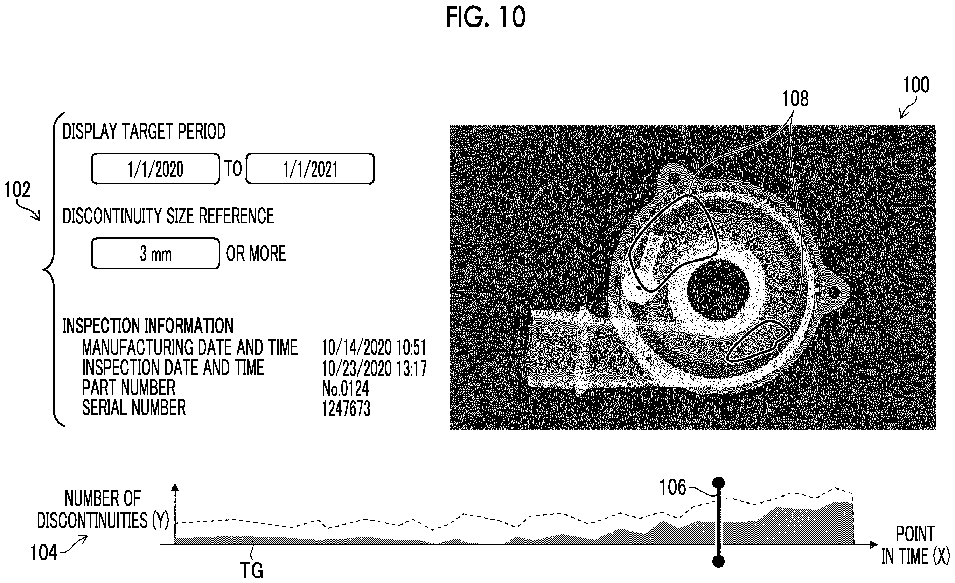

Fig. 10 is a diagram showing a third display mode. In addition, the portions already described in Figs. 6 and 7 are denoted by the same references, and the description thereof will not be repeated.

In the third display mode, the radiation image 100, the occurrence position distribution 108 of discontinuities displayed to be superimposed on the radiation image 100, the detailed information display 102, and the timeline bar 104 having the pointer 106 are displayed.

In the detailed information display 102 according to the present display mode, a reference value of the discontinuity information displayed in the occurrence position distribution of discontinuities is shown. In a case shown in Fig. 10, a discontinuity size reference value is set to 3 mm or more. Therefore, the occurrence position distribution 108 to be displayed is displayed based on the discontinuity of 3 mm or more. For example, in a case where the interpreter determines the defect/non-defect and wants to check the occurrence distribution of the discontinuity of 3 mm or more, the present display mode can efficiently and accurately check the occurrence distribution.

In addition, the number of discontinuities TG displayed together on the timeline bar 104 indicates the number of discontinuities of 3 mm or more. Accordingly, the user can visually ascertain the number of discontinuities of 3 mm or more.

As described above, in the present display mode, since the occurrence position distribution is displayed according to the set reference value, the user can efficiently and accurately check the occurrence distribution of the discontinuities.

### <Fourth display mode>

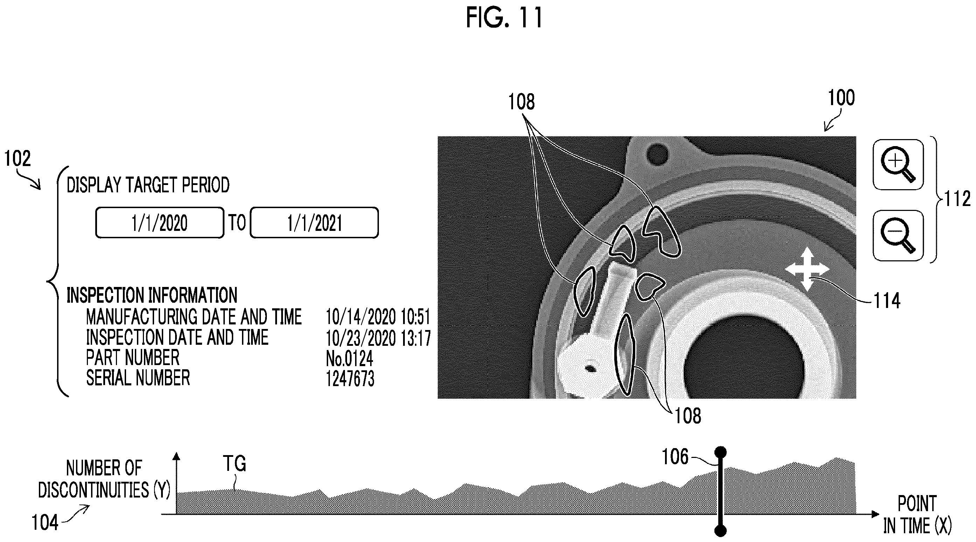

Fig. 11 is a diagram showing a fourth display mode. In addition, the portions already described in Figs. 6 and 7 are denoted by the same references, and the description thereof will not be repeated.

In the fourth display mode, the radiation image 100, the occurrence position distribution 108 of discontinuities displayed to be superimposed on the radiation image 100, the detailed information display 102, the timeline bar 104 having the pointer 106, a movement cursor 114, and an enlargement/reduction icon 112 are displayed.

The movement cursor 114 can move the occurrence position distribution 108 and the radiation image 100 by being selected by the user. The user can move the occurrence position distribution 108 and the radiation image 100 to any position and display the occurrence position distribution 108 and the radiation image 100 by selecting the movement cursor 114.

The enlargement/reduction icon 112 performs enlargement and reduction of the occurrence position distribution 108 and the radiation image 100. By selecting the "plus (+)" display of the enlargement/reduction icon 112, the enlargement of the occurrence position distribution 108 and the radiation image 100 is performed. Meanwhile, by selecting the "minus (-)" display of the enlargement/reduction icon 112, the reduction of the occurrence position distribution 108 and the radiation image 100 is performed.

As described above, in the present display mode, the user can change the radiation image 100 and the occurrence position distribution 108 to any position and size by using the movement cursor 114 and the enlargement/reduction icon 112. As a result, the user can efficiently and accurately check the occurrence distribution of the discontinuities.

### <Fifth display mode>

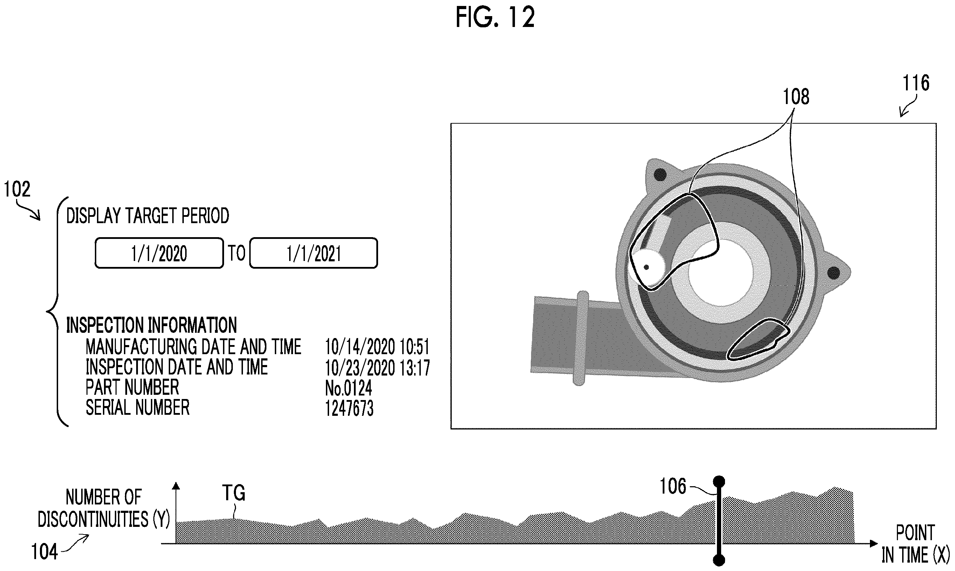

Fig. 12 is a diagram showing a fifth display mode. In addition, the portions already described in Figs. 6 and 7 are denoted by the same references, and the description thereof will not be repeated.

In the fifth display mode, the radiation image 100, the occurrence position distribution 108 of discontinuities displayed to be superimposed on a design drawing 116, the detailed information display 102, and the timeline bar 104 having the pointer 106 are displayed.

In the present display mode, the occurrence position distribution 108 is displayed on the design drawing 116 of the component instead of the radiation image 100. The radiation image 100 may not be clearly captured depending on components, portions, and the like. In such a case, the occurrence position distribution 108 is displayed to be superimposed on the design drawing 116 of the component to be displayed, so that the occurrence distribution of the discontinuities can be efficiently and accurately checked. In the above-described specific example, the specific example in which the design drawing 116 is used instead of the radiation image 100 has been described, but an image related to another component may be used instead of the radiation image 100. For example, a superimposition image obtained by superimposing a plurality of the radiation images subjected to registration may be used instead of the radiation image 100.

In this way, by displaying the occurrence position distribution 108 to be superimposed on the design drawing 116 of the component instead of the radiation image 100, various display modes can be employed. As a result, the user can efficiently and accurately check the occurrence position distribution of the discontinuities.

### <Calculation Method and Display Mode of Occurrence Position Distribution>

Next, the calculation of the occurrence position distribution and the display mode (schematic diagram) of the occurrence position distribution will be described. Various methods are employed for the calculation of the occurrence position distribution and the display mode of the occurrence position distribution.

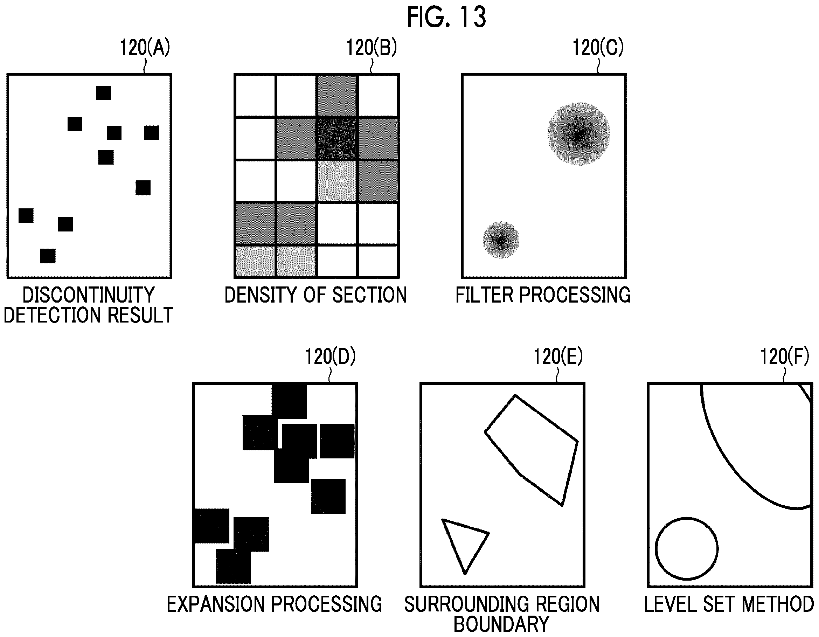

Fig. 13 is a diagram illustrating the display mode of the occurrence position distribution.

In a case where the detected discontinuities are individually displayed as they are in displaying the occurrence position distribution, the occurrence position distribution of the discontinuities may be difficult to understand. Reference 120(A) of Fig. 13 shows a schematic diagram in a case where a detection result of discontinuities is displayed as it is. In this way, in a case where the detection result of the discontinuities is simply displayed, it may be difficult to accurately ascertain the occurrence position distribution. Therefore, the individual detected discontinuities may be aggregated and displayed as an occurrence region.

Here, the display of the occurrence region can employ, for example, a first display method and a second display method described below.

In the first display method, a frequency is displayed in density (continuous display method). The first display method is, for example, an aspect in which for an image on which discontinuity detection has been performed, the image is divided into sections and the frequency of the sections is converted into the density and displayed (for example, see reference 120(B)). In addition, for example, there is an aspect in which for an image on which discontinuity detection has been performed, the image is divided in units of pixels, and a filter such as a Gaussian filter is subjected to a convolution operation and displayed (see reference 120(C)).

In the second display method, a boundary of a region is displayed (discrete display method). For example, in the second display method, there is a display mode in which for an image on which discontinuity detection has been performed, discontinuity regions are grouped together by expansion processing of discontinuities or the like (see reference 120(D)). In addition, for example, there is an aspect in which a group including discontinuities within a specified distance is surrounded by a polygon and further, for ease of viewing, is displayed by being surrounded by a convex hull using a QuickHull technique or the like (see reference 120(E)). In addition, for example, there is an aspect in which a boundary line is obtained using a level set method and is displayed (see reference 120(F)).

As described above, the calculation method of the occurrence position distribution and the display mode of the occurrence position distribution in the present invention can employ various modes.

### <Acquisition of Discontinuity Information>

Next, the acquisition of the discontinuity information (see Fig. 2) stored in the discontinuity information DB 22 will be described.

Fig. 14 is a block diagram showing a defect inspection device that acquires the discontinuity information. In Fig. 14, the information processing apparatus 10 connected from the outside to the discontinuity information DB 22 and the display unit 26 connected to the information processing apparatus 10 are shown.

The defect inspection device 510 is a device that detects and displays a discontinuity (defect candidate) that is a candidate for a defect from an image obtained by imaging an industrial product to be inspected (an inspection object: corresponding to a component), and is used to support a diagnosis of a defect of the inspection object by an interpreter. As shown in Fig. 14, the defect inspection device 510 according to the present embodiment comprises a controller 512, an operation unit 514, an input/output interface (hereinafter, referred to as an I/F) 516, a display unit 526, a buffer memory 520, the image processing unit 522, and the discontinuity information DB 22.

The controller 512 includes a central processing unit (CPU) that controls operations of units of the defect inspection device 510. The controller 512 receives an operation input from the interpreter through the operation unit 514 and transmits a control signal corresponding to the operation input to each unit of the defect inspection device 510 to control the operation of each unit.

The operation unit 514 is an input device that receives the operation input from the interpreter, and includes a keyboard for inputting characters and a pointing device (a mouse, a trackball, or the like) for operating a pointer, an icon, and the like displayed on the display unit 526. In addition, a touch panel may be provided on a surface of the display unit 526 as the operation unit 514, instead of the means listed above or in addition to the means listed above.

The I/F 516 is means for performing communication with an external device via a network NW. As a method of transmitting and receiving data between the defect inspection device 510 and the external device, wired communication (for example, local area network (LAN), wide area network (WAN), Internet connection, or the like) or wireless communication (for example, LAN, WAN, internet connection, or the like) can be used.

The defect inspection device 510 can receive the input of inspection object imaging data D100 including captured image data (for example, the radiation image 100) of an inspection object OBJ imaged by the imaging system 500 via the I/F 516. In addition, a method of inputting the inspection object imaging data D100 from the imaging system 500 to the defect inspection device 510 is not limited to the communication via the network NW listed above. For example, the defect inspection device 510 and the imaging system 500 may be connected to each other by a universal serial bus (USB) cable, Bluetooth (registered trademark), infrared communication, or the like. The inspection object imaging data D100 may be stored in a memory card attachable to and detachable from the defect inspection device 510 and readable by the defect inspection device 510, and the image data may be input to the defect inspection device 510 via the memory card.

Further, the defect inspection device 510 can communicate with a product database (product DB) 200 via the network NW. Product data D200 for each component that can be an inspection target is stored in a product DB 200. The controller 512 can search for and read out inspection object specifying information for specifying the inspection object from the inspection object imaging data of the inspection object OBJ acquired from the imaging system 500 and can acquire the product data D200 corresponding to the read-out inspection object specifying information from the product DB 200. By using the product data D200, it is possible to detect a discontinuity (defect candidate) according to a type or a feature of the inspection object OBJ.

The product DB 200 may be installed on the network NW as in the present embodiment so that the manufacturer or the like may be able to update the product data D200, or the product DB 200 may be provided in the defect inspection device 510.

The display unit 526 is a device for displaying an image. As the display unit 526, for example, a liquid crystal monitor (see Fig. 5) can be used.

The buffer memory 520 is used as a region for temporarily storing a work region of the controller 512 and image data to be output to the display unit 526.

The discontinuity information DB 22 stores the discontinuity information acquired as described above in association with each component. In addition, the discontinuity information DB 22 stores the inspection object imaging data D100 and the product data D200 in association with each component.

The image processing unit 522 reads out the captured image data of the inspection object OBJ from the inspection object imaging data D100 and performs image processing on the captured image data to detect the discontinuity. The image processing unit 522 outputs the captured image data and discontinuity detection result information(detection result) indicating a detection result (calculation result of the feature) of the detected discontinuity to the buffer memory 520. The controller 512 creates a display image in which the detected discontinuity detection result information (detection result) is added on the captured image data by using data output to the buffer memory 520, and displays the display image on the display unit 526. As a result, the interpreter can interpret the image displayed on the display unit 526 and perform the inspection of the inspection object OBJ. In the present example, a case where the detection of the discontinuity is performed by the image processing unit 522 has been described, but the detection of the discontinuity is not limited to this example. For example, the detection of the discontinuity may be performed by the interpreter observing the radiation image displayed on the display unit 26.

The interpreter can input a diagnosis result, such as a defect or a non-defect, to each piece of the discontinuity information added to the image displayed on the display unit 526 via the operation unit 514. In addition, for example, the interpreter may input a diagnosis result such as "immediately replace the inspection object OBJ with a new one", "observe the progress (re-inspect after a days)", or "leave it (not a defect)".

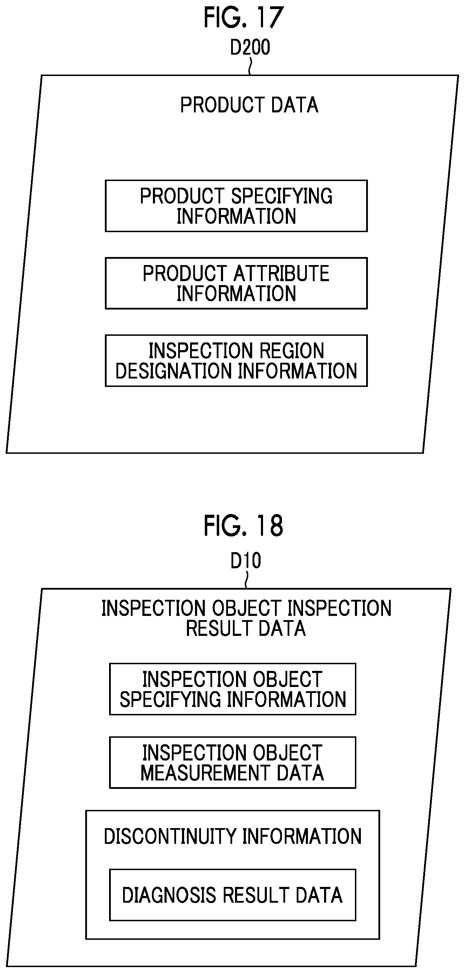

The controller 512 creates inspection object inspection result data D10 (see Fig. 18) including the diagnosis result data and stores the inspection object inspection result data D10 in the discontinuity information DB 22.

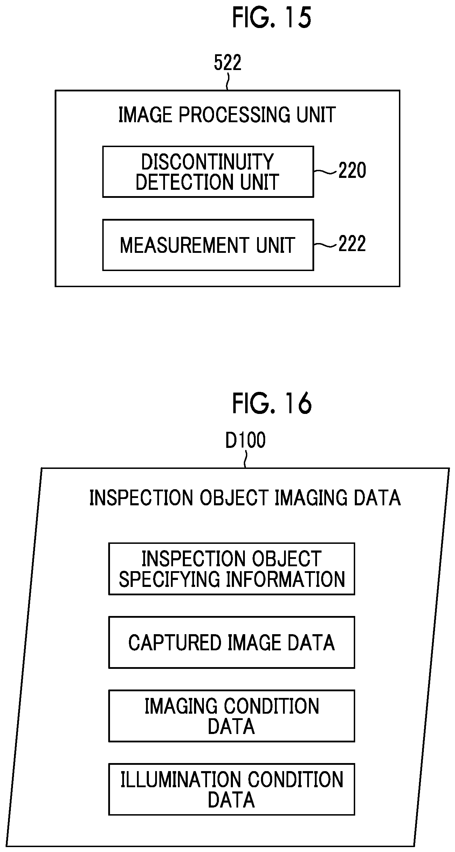

Fig. 15 is a block diagram showing an example of the image processing unit. As shown in Fig. 15, the image processing unit 522 comprises a discontinuity detection unit 220 and a measurement unit 222.

The discontinuity detection unit 220 detects discontinuities (for example, cracks, abrasion, rust, Porosity, Gas Hole, FMMD, and the like) of the inspection object OBJ by performing image processing (for example, color conversion processing, monochrome conversion processing, edge enhancement processing, conversion processing into three-dimensional data, and the like) on the captured image data to detect a change in color or the like of the inspection object OBJ. As a result, a position and a shape of the discontinuity are specified.

For example, product image data including an image of a (new) product in which the same discontinuity of the inspection object OBJ is not detected may be included in the product data D200, and the discontinuity may be detected by comparing the product image data with the captured image data of the inspection object OBJ. In addition, for example, the discontinuity detection unit 220 may be configured of a detector subjected to known machine learning.

The measurement unit 222 measures dimensions of each part of the inspection object OBJ based on the captured image data of the inspection object OBJ and imaging condition data. The measurement unit 222 measures the size of the inspection object OBJ based on, for example, the imaging condition data, such as a distance between a camera and the inspection object OBJ during imaging, a focal length, and a zoom magnification, and a size of the inspection object OBJ in the captured image data. The measurement unit 222 calculates a size (for example, a maximum dimension, a minimum dimension, a depth of crack, and an angle) of the discontinuity by using the measured size of the inspection object OBJ, the size of the inspection object OBJ in the captured image data, and the size of the discontinuity. The size of the inspection object OBJ may be acquired via the product data D200.

Further, the measurement unit 222 measures a thickness for each position of the inspection object OBJ using the dimensions of each part of the inspection object OBJ and information indicating, for example, a reflectivity and a transmittance (transmission attenuation) of irradiation light during imaging of the inspection object OBJ. The thickness may be measured by the imaging system 500 during imaging and may be included in the inspection object imaging data D100.

Fig. 16 is a block diagram showing an example of the inspection object imaging data. As shown in Fig. 16, the inspection object imaging data D100 includes the inspection object specifying information, the captured image data, the imaging condition data, and illumination condition data.

The inspection object specifying information is information for specifying the inspection object OBJ, and includes, for example, information indicating a product name, a product number, a part number, a serial number, a manufacturer name, and a technical classification of the inspection object OBJ.

The captured image data is image data (for example, a radiation image or a visible light image) obtained by imaging the inspection object OBJ.

The imaging condition data is stored for each captured image data of the inspection object OBJ, and includes information indicating an imaging date and time, an imaging target portion, a distance between the inspection object OBJ and the camera during imaging, and an angle with respect to the camera for each captured image data.

The illumination condition data includes information indicating a type of radiation used for imaging the inspection object OBJ (for example, X-rays (radiation), visible rays, transmission rays, or reflection rays), an irradiation intensity, and an irradiation angle.

Fig. 17 is a block diagram showing an example of the product data. As shown in Fig. 17, the product information includes product specifying information, product attribute information, and inspection region designation information. The product data D200 may be recorded in the discontinuity information DB 22 in association with the inspection object imaging data D100 and the inspection object inspection result data D10 via the inspection object specifying information and the product specifying information and, or may be acquired from the product DB 200 each time the defect is inspected.

The product specifying information is information for specifying the product, and includes, for example, information indicating a product name, a product number, a part number, a serial number, a manufacturer name, and a technical classification.

The product attribute information includes, for example, information indicating a material and a dimension of each part of the product, and an application of the product. The information indicating the application of the product includes, for example, information on a name, a type, a processing state, and an attachment method (for example, joints, welds, screwing, fitting, and soldering) of a device to which the product is attached, or the like. In addition, the product attribute information includes defect (or discontinuity) occurrence information. The defect occurrence information includes, for example, information of at least one of a past inspection date and time, a material of the inspection object OBJ, a type (for example, a foreign substance, a crack, or the like), a shape, a size, a depth, or an occurrence portion (portion coordinates, a material thickness, a processing state (for example, joints, welds, or the like)) of the defect that has occurred in the past, or the captured image of the defect.

In a case where the discontinuity is detected from the inspection object OBJ, the image processing unit 522 can increase a detection accuracy of the discontinuity (for example, reduce a minimum size (a threshold value of a size) of a scratch or the like detected as the discontinuity, or a threshold value of a depth of a crack) for an inspection region designated by the inspection region designation information. In addition, in a case where the image of the inspection object OBJ and the image of the discontinuity are displayed on the display unit 526, a mark or the like for identifying the captured image data of the inspection region and the discontinuity detected from a detection target region may be added, or processing of emphasizing these may be performed.

In a case of a product having a plurality of applications, the inspection region designation information may be created for each application of the product (for example, for each type of a device to which the product is attached and for each attachment location), and the detection of the discontinuity may be performed by using the inspection region designation information corresponding to the designated application.

In addition, in a case where there is no product data in which the product name or the product number matches, product data of a product having a similar technical classification may be acquired and used for the image processing.

Fig. 18 is a block diagram showing an example of the inspection object inspection result data. As shown in Fig. 18, the inspection object inspection result data D10 includes inspection object measurement information, discontinuity information, and diagnosis result information in addition to the inspection object specifying information. The inspection object inspection result data D10 is recorded in the discontinuity information database 22 in association with the inspection object imaging data D100 via the inspection object specifying information.

The inspection object measurement data includes measurement results of a size of the inspection object OBJ and a thickness of the inspection object OBJ for each position by the measurement unit 222.

The discontinuity information includes information indicating features of the discontinuity (for example, a discontinuity ID, a type of the discontinuity, a position and a size of the discontinuity, a change amount of a thickness, and the like). The information indicating the position of the discontinuity can be represented by, for example, coordinates on a coordinate system (for example, a three-dimensional orthogonal coordinate system, a polar coordinate system, a cylindrical coordinate system, or the like) set according to the shape of the inspection object OBJ. The information indicating the type of the discontinuity is information created based on, for example, the shape or the like of the discontinuity detected from the image. Specific examples of the type of the discontinuity include Porosity, Gas Hole, FMMD, a granular defect, a stain-like defect, and a crack-like defect. In addition, the discontinuity information includes the diagnosis result data. The diagnosis result data includes the inspection date and time and information that is additionally input by the interpreter with respect to the discontinuity. The diagnosis result data includes, for example, information indicating a diagnosis result input by the interpreter, such as a defect or a non-defect.

The inspection object inspection result data D10 may include a part of the inspection object imaging data D100 and the product data D200.

In addition, the inspection object inspection result data D10 may be transmitted to the product DB 200 to be accumulated therein, and the inspection region designation information of the product data D200 may be updated by using results obtained by analyzing the discontinuity information and the diagnosis result data included in the inspection object inspection result data D10.

Next, the imaging system 500 for capturing the image of the inspection object OBJ will be described. Fig. 19 is a block diagram showing an example of the imaging system.

The imaging system 500 is used to image the inspection object OBJ placed in an imaging room 513, and comprises an imaging controller 502, an imaging operation unit 504, an image recording unit 506, a camera 508, and radiation sources 509 and 511 as shown in Fig. 19.

The imaging controller 502 includes a central processing unit (CPU) that controls an operation of each unit of the imaging system 500. The imaging controller 502 receives an operation input from an operator (imaging technician) via the imaging operation unit 504, and transmits a control signal corresponding to the operation input to each unit of the imaging system 500 to control the operation of each unit.

The imaging operation unit 504 is an input device that receives the operation input from the operator, and includes a keyboard for inputting characters and a pointing device (a mouse, a trackball, or the like) for operating a pointer, an icon, and the like displayed on the display unit 526. The operator can perform, through the imaging operation unit 504, an input of information regarding the inspection object OBJ, an input of an instruction to execute imaging to the camera 508 (including settings for imaging conditions such as an exposure time, a focal length, and a stop, an imaging angle, an imaging portion, and the like), an input of an instruction of radiation irradiation to the radiation sources 509 and 511 (including settings for an irradiation start time, an irradiation duration, an irradiation angle, an irradiation intensity, and the like), and an input of an instruction to record the acquired image data in the image recording unit 506.

The image recording unit 506 records image data (light-receiving image) of the inspection object OBJ imaged by the camera 508. The image recording unit 506 records information for specifying the inspection object OBJ in association with the image data.

The camera 508 and the radiation sources 509 and 511 are disposed inside the imaging room 513. The radiation sources 509 and 511 are, for example, X-ray sources, and a partition wall between the imaging room 513 and the outside and an entrance are protected from X-rays by X-ray protective materials (for example, lead, concrete, or the like). In a case where the inspection object OBJ is irradiated with visible light for imaging, it is not necessary to use the imaging room 513 with protection.

The radiation sources 509 and 511 irradiate the inspection object OBJ placed in the imaging room 513 with radiation in response to an instruction from the imaging controller 502.

The camera 508 receives radiation emitted to the inspection object OBJ from the radiation source 509 and reflected by the inspection object OBJ or radiation emitted to the inspection object OBJ from the radiation source 511 and transmitted through the inspection object OBJ and images the inspection object OBJ, in response to an instruction to execute imaging from the imaging controller 502. The inspection object OBJ is held in the imaging room 513 by a holding member (for example, a manipulator, a mounting table, or a movable mounting table) which is not shown, and a distance and an angle of the inspection object OBJ with respect to the camera 508 and the radiation sources 509 and 511 can be adjusted. The operator can control relative positions between the inspection object OBJ, the camera 508, and the radiation sources 509 and 511 via the imaging controller 502, and can image a desired portion of the inspection object OBJ.

The radiation sources 509 and 511 end the irradiation of the inspection object OBJ with the radiation in synchronization with the end of the execution of the imaging by the camera 508.

In the example shown in Fig. 19, the camera 508 is disposed inside the imaging room 513, but the camera 508 may be disposed outside as long as it can image the inspection object OBJ in the imaging room 513.

In addition, in the example shown in Fig. 19, one camera 508 and two radiation sources 509 and 511 are provided, but the number of the cameras and the radiation sources is not limited thereto. For example, a plurality of cameras and a plurality of radiation sources may be provided, or one camera and one radiation source may be provided.

### <Others>

In the above-described embodiment, hardware structures of processing units (such as the display selection instruction receiving unit 30, the discontinuity information acquisition unit 32, the occurrence position distribution acquisition unit 34, and the display controller 36) that execute various kinds of processing are various processors as described below. The various processors include a central processing unit (CPU) that is a general-purpose processor functioning as various processing units by executing software (program), a programmable logic device (PLD) such as a field programmable gate array (FPGA) that is a processor having a circuit configuration changeable after manufacture, a dedicated electric circuit such as an application specific integrated circuit (ASIC) that is a processor having a circuit configuration dedicatedly designed to execute specific processing, and the like.

One processing unit may be configured of one of the various processors or may be configured of two or more processors of the same type or different types (for example, a plurality of FPGAs or a combination of a CPU and an FPGA). In addition, a plurality of processing units may be configured of one processor. As an example of configuring the plurality of processing units by one processor, first, there is a form in which one processor is configured of a combination of one or more CPUs and software, as typified by a computer such as a client or a server, and the one processor functions as the plurality of processing units. Second, as represented by a system on chip (SoC) or the like, a form of using a processor that implements, by one integrated circuit (IC) chip, functions of the entire system including the plurality of processing units is included. As described above, the various processing units are configured using one or more of the above various processors as a hardware structure.

Further, as the hardware structure of the various processors, more specifically, an electric circuit (circuitry) in which circuit elements such as semiconductor elements are combined may be used.

Each of the configurations and functions described above can be appropriately realized by using any hardware, software, or a combination of both. For example, the present invention can also be applied to a program for causing a computer to execute the above-described processing steps (processing procedures), a computer-readable recording medium (non-transitory recording medium) on which such a program is recorded, or a computer on which such a program can be installed.

Although examples of the present invention have been described above, it goes without saying that the present invention is not limited to the above-described embodiment and various modifications can be made without departing from the scope of the present invention.

### Explanation of References

8: bus
10: information processing apparatus
12: input/output interface
14: processor
16: memory
18: inspection result display program
22: information database
24: operation unit
26: display unit
30: display selection instruction receiving unit
32: information acquisition unit
34: occurrence position distribution acquisition unit
36: display controller
100: radiation image
102: detailed information display
104: timeline bar
106: pointer
108: occurrence position distribution
110: type display
112: enlargement/reduction icon
114: movement cursor
116: design drawing

## Claims

1. An information processing apparatus comprising a processor,
wherein the processor is configured to:
receive a display selection instruction from an operation unit operated by a user;
acquire discontinuity information that is information related to a discontinuity of a component and includes positional information of the discontinuity on the component;
acquire an occurrence position distribution of the discontinuity based on the discontinuity information; and
display the occurrence position distribution on an image of the component in response to the display selection instruction.

2. The information processing apparatus according to claim 1,
wherein the display selection instruction gives an instruction for positions of arrangement of a plurality of the components, and the occurrence position distribution corresponding to the positions of the arrangement is displayed.

3. The information processing apparatus according to claim 2,
wherein the arrangement has a component in time.

4. The information processing apparatus according to claim 3,
wherein the time is related to manufacturing or inspection of the component.

5. The information processing apparatus according to any one of claims 1 to 4,
wherein the discontinuity is detected by transmission of radiation.

6. The information processing apparatus according to any one of claims 1 to 5,
wherein the processor is configured to display the discontinuity information in relation to the occurrence position distribution.

7. The information processing apparatus according to any one of claims 1 to 6,
wherein the processor is configured to perform a user interface display indicating a point in time of manufacturing of the component or inspection of the component on a display unit, and
the display selection instruction is received by the user interface display and is composed of the point in time of manufacturing of the component or inspection of the component.

8. The information processing apparatus according to any one of claims 1 to 6,
wherein the processor is configured to perform a user interface display indicating a manufacturing number of the component on a display unit, and
the display selection instruction is received by the user interface display and is composed of the manufacturing number.

9. The information processing apparatus according to claim 7 or 8,
wherein the processor is configured to also display the discontinuity information on the user interface display.

10. The information processing apparatus according to claim 9,
wherein the discontinuity information displayed on the user interface display is the number of discontinuities or a total area of the discontinuities.

11. The information processing apparatus according to claim 10,
wherein the discontinuity information is displayed on the user interface display according to a set reference value.

12. The information processing apparatus according to any one of claims 7 to 11,
wherein the discontinuity information includes information on a type of the discontinuity, and
the user interface display displays the discontinuity information for each type of the discontinuity.

13. The information processing apparatus according to any one of claims 7 to 12,
wherein the display selection instruction selects a period on the user interface display, and
the occurrence position distribution corresponding to the period is displayed on the image.

14. The information processing apparatus according to any one of claims 1 to 13,
wherein the display of the occurrence position distribution is movable.

15. The information processing apparatus according to any one of claims 1 to 14,
wherein the display of the occurrence position distribution is enlargeable or reducible.

16. The information processing apparatus according to any one of claims 1 to 15,
wherein the image is a radiation image of the component, a plurality of the radiation images subjected to registration, or a design drawing of the component.

17. The information processing apparatus according to any one of claims 1 to 16,
wherein the display selection instruction is a selection of the component to be displayed.

18. An information processing method for an information processing apparatus including a processor, the method causing the processor to execute:
a step of receiving a display selection instruction from an operation unit operated by a user;
a step of acquiring discontinuity information that is information related to a discontinuity of a component and includes positional information of the discontinuity on the component;
a step of acquiring an occurrence position distribution of the discontinuity based on the discontinuity information; and
a step of displaying the occurrence position distribution on an image of the component in response to the display selection instruction.

19. A program for causing an information processing apparatus including a processor to execute an information processing method, the program causing the processor to execute:
a step of receiving a display selection instruction from an operation unit operated by a user;
a step of acquiring discontinuity information that is information related to a discontinuity of a component and includes positional information of the discontinuity on the component;
a step of acquiring an occurrence position distribution of the discontinuity based on the discontinuity information; and
a step of displaying the occurrence position distribution on an image of the component in response to the display selection instruction.

20. A non-transitory computer-readable recording medium on which the program according to claim 19 is recorded.

## Amended claims

### Amended claims under Art. 19.1 PCT

1. An information processing apparatus comprising a processor, wherein the processor is configured to:
receive a display selection instruction from an operation unit operated by a user;
acquire discontinuity information that is information related to a discontinuity of a component and includes positional information of the discontinuity on the component;
acquire an occurrence position distribution of the discontinuity based on the discontinuity information; and
display the occurrence position distribution on an image of the component in response to the display selection instruction.

2. The information processing apparatus according to claim 1,
wherein the display selection instruction gives an instruction for positions of arrangement of a plurality of the components, and the occurrence position distribution corresponding to the positions of the arrangement is displayed.

3. The information processing apparatus according to claim 2,
wherein the arrangement has a component in time.

4. The information processing apparatus according to claim 3,
wherein the time is related to manufacturing or inspection of the component.

5. The information processing apparatus according to any one of claims 1 to 4,
wherein the discontinuity is detected by transmission of radiation.

6. The information processing apparatus according to any one of claims 1 to 5,
wherein the processor is configured to display the discontinuity information in relation to the occurrence position distribution.

7. The information processing apparatus according to any one of claims 1 to 6,
wherein the processor is configured to perform a user interface display indicating a point in time of manufacturing of the component or inspection of the component on a display unit, and
the display selection instruction is received by the user interface display and is composed of the point in time of manufacturing of the component or inspection of the component.

8. The information processing apparatus according to any one of claims 1 to 6,
wherein the processor is configured to perform a user interface display indicating a manufacturing number of the component on a display unit, and
the display selection instruction is received by the user interface display and is composed of the manufacturing number.

9. The information processing apparatus according to claim 7 or 8,
wherein the processor is configured to also display the discontinuity information on the user interface display.

10. The information processing apparatus according to claim 9,
wherein the discontinuity information displayed on the user interface display is the number of discontinuities or a total area of the discontinuities.

11. The information processing apparatus according to claim 10,
wherein the discontinuity information is displayed on the user interface display according to a set reference value.

12. The information processing apparatus according to any one of claims 7 to 11,
wherein the discontinuity information includes information on a type of the discontinuity, and
the user interface display displays the discontinuity information for each type of the discontinuity.

13. The information processing apparatus according to any one of claims 7 to 12,
wherein the display selection instruction selects a period on the user interface display, and
the occurrence position distribution corresponding to the period is displayed on the image.

14. (Amended) The information processing apparatus according to any one of claims 7 to 13, wherein the user interface display is a timeline bar.

15. (Amended) The information processing apparatus according to any one of claims 1 to 14, wherein the display of the occurrence position distribution is movable.

16. (Amended) The information processing apparatus according to any one of claims 1 to 15, wherein the display of the occurrence position distribution is enlargeable or reducible.

17. (Amended) The information processing apparatus according to any one of claims 1 to 16, wherein the image is a radiation image of the component, a plurality of the radiation images subjected to registration, or a design drawing of the component.

18. (Amended) The information processing apparatus according to any one of claims 1 to 17, wherein the display selection instruction is a selection of the component to be displayed.

19. (Amended) The information processing apparatus according to any one of claims 1 to 18, wherein the occurrence position distribution is displayed as an occurrence region.

20. (Amended) The information processing apparatus according to claim 19, wherein the occurrence region is a region in which positions of respective discontinuities are aggregated based on the discontinuity information.

21. (Added) The information processing apparatus according to claim 19, wherein the occurrence region is a region in which occurrence frequency of discontinuities are displayed in density based on the discontinuity information.

22. (Added) The information processing apparatus according to claim 19, wherein the occurrence region is a region formed from a boundary line based on the discontinuity information.

23. (Added) An information processing method for an information processing apparatus including a processor, the method causing the processor to execute:
a step of receiving a display selection instruction from an operation unit operated by a user;
a step of acquiring discontinuity information that is information related to a discontinuity of a component and includes positional information of the discontinuity on the component;
a step of acquiring an occurrence position distribution of the discontinuity based on the discontinuity information; and
a step of displaying the occurrence position distribution on an image of the component in response to the display selection instruction.

24. (Added) A program for causing an information processing apparatus including a processor to execute an information processing method, the program causing the processor to execute:
a step of receiving a display selection instruction from an operation unit operated by a user;
a step of acquiring discontinuity information that is information related to a discontinuity of a component and includes positional information of the discontinuity on the component;
a step of acquiring an occurrence position distribution of the discontinuity based on the discontinuity information; and
a step of displaying the occurrence position distribution on an image of the component in response to the display selection instruction.

25. (Added) A non-transitory computer-readable recording medium on which the program according to claim 24 is recorded.

Statement under Art. 19.1 PCT
Claim 14 newly recites an invention based on matters written in paragraphs [0052] to [0062] in the description as filed. Original claims 14-17 are amended to renumber their claim numbers to be claims 15 to 18 due to the addition of claim 14. Claim 19 newly recites an invention based on matters written in paragraphs [0087] and [0088] in the description as filed. Claim 20 newly recites an invention based on the paragraph [0087] in the description and figure 13 as filed. Claim 21 newly recites an invention based on matters written in paragraph [0089] in the description and figure 13 as filed. Claim 22 newly recites an invention based on matters written in paragraph [0090] in the description and figure 13 as filed. New claims 23-25 are prepared by renumbering claim numbers of original claims 19-22 due to the addition of claims 19-22.
